(19) Europäisches Patentamt / European Patent Office / Office européen des brevets

(11) **EP 3 510 612 B1**

(12) **EUROPEAN PATENT SPECIFICATION**

(45) Date of publication and mention
of the grant of the patent:
**27.01.2021  Bulletin 2021/04**

(21) Application number: **17767881.0**

(22) Date of filing: **06.09.2017**

(51) Int Cl.:
*H01F 7/16* *(2006.01)*       *H01F 7/122* *(2006.01)*
*H01F 7/14* *(2006.01)*       *A61M 39/28* *(2006.01)*
*F16K 7/04* *(2006.01)*

(86) International application number:
**PCT/GB2017/052592**

(87) International publication number:
**WO 2018/046909 (15.03.2018 Gazette 2018/11)**

(54) **GAS SUPPLY CONTROL SYSTEM COMPRISING AN ELECTROMAGNETIC ACTUATOR**

GASZUFUHRREGELUNG MIT ELEKTROMAGNETISCHEM STELLGLIED

SYSTÈME D'ALIMENTATION EN GAZ COMPRENANT UN ACTIONNEUR ÉLECTROMAGNÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.09.2016  GB 201615379
28.02.2017  GB 201703228**

(43) Date of publication of application:
**17.07.2019  Bulletin 2019/29**

(73) Proprietor: **Camcon Medical Limited
Cambridge, Cambridgeshire CB24 9NL (GB)**

(72) Inventors:
• **WYGNANSKI, Wladyslaw
Cambridge
Cambridgeshire CB22 5JR (GB)**
• **MCDOUALL, Christopher
Cambridge
Cambridgeshire CB3 0NE (GB)**

(74) Representative: **Sharrock, Daniel John et al
Nash Matthews LLP
24 Hills Road
Cambridge CB2 1JP (GB)**

(56) References cited:
EP-A1- 0 170 894       EP-A1- 2 945 264
DE-A1- 19 945 262      FR-A1- 2 865 312
GB-A- 1 590 373        US-A- 4 533 890
US-A- 4 829 947        US-A- 4 910 487
US-A- 5 351 934        US-B1- 6 173 711

## Description

Field of the Invention

The present invention relates to gas supply control systems comprising electromagnetic actuators, and more particularly but not exclusively to electromagnetic actuators that are able to operate silently. It also concerns combination of a valve with an electromagnetic actuator.

Background of the Invention

In known electromagnetic actuators, an armature including a permanent magnet is switchable between two stable rest positions using a pair of coils. Some examples of actuators of this form are described in GB-A-2466102.

Further examples of electromagnetic actuators are disclosed in US4,829,947, US5,351,934, FR2865312, US4,533,890, DE19945262, EP0170894, US4,910,487, EP2945264 and GB1590373 US6173711B1 discloses a gas supply control system which includes a pressure sensor for respiratory masks.

Pinch valves are able to control the flow of a fluid through tubing without the valve entering the flowpath. The valve controls the fluid flow by pinching the tubing. Pinch valves are therefore particularly suitable for applications where contamination of the fluid is undesirable, such as medical applications, chemical process engineering and food technology for example.

Known electromagnetically operated pinch valves use solenoid type actuators. Such actuators are switchable between positions in which the tubing is either pinched or unpinched. A solenoid actuator is biased towards one position and when energised, it moves to and is retained in the other position. When the energising current is removed, the actuator then reverts to its original position, under the influence of the biasing force.

Summary of the Invention

The present invention provides a gas supply control system according to claim 1.

Accordingly, the armature may not contact a coil core (and preferably is not brought into contact with any other part of the actuator) as it moves into each rest position. This avoids the generation of a clicking noise as the armature switches into either rest position. In known bistable actuators, an armature is accelerated into contact with a coil core in each stable rest position, causing a loud clicking noise to be produced. In many applications at which the present actuator is directed, such as medical environments, frequent generation of clicking sounds may be undesirable.

The actuator may have only two stable rest positions and therefore constitute a bistable actuator. In some embodiments, there may be two end-of-travel positions which are stable rest positions. In some actuators embodying the invention, there may also be one or more further stable rest positions located between the two end-of-travel positions which may be selected by suitable control of the actuator.

The actuator preferably includes two resilient components, wherein one of the resilient components is compressed by movement of the armature towards each stable rest position (or more particularly, in some embodiments, during the final part of the movement of the armature towards and into each stable rest position).

The resilient component may be able to store potential energy as the armature moves into each of its first and second rest positions. When the armature is switched towards its other position, this potential energy is released and acts to accelerate the armature towards the other position. The resilient component may be in the form of a single resilient part or alternatively it may be an assembly of parts.

In a preferred embodiment, the actuator includes a push rod which extends along a central axis of the actuator and is moveable relative to the coils along the central axis. Preferably, the armature is axially slideable along the push rod. The actuator configuration may permit relative movement between the armature and the push rod in order to reduce the impulse exerted by the push rod in response to forces acting on the armature. This may be beneficial when the push rod is acting on a viscous fluid for example, as smoother application of force to such a fluid may provide a smoother switching action.

More preferably, one end of each resilient component may be fixed in position relative to the push rod so as to be unable to move relative thereto. For example, it may be mounted in a fixed position on the push rod or bear against another component which is fixed in position relative to the push rod. Alternatively, one end of each resilient component may bear against an internal fixed surface of the actuator. The armature may be arranged to exert a compressive force against the other end of one of the resilient components by sliding along the push rod as it moves towards a stable rest position. This arrangement may reduce the impulse exerted by the push rod in response to forces acting on the armature, as the resilient component may act to absorb some of the kinetic energy of the armature.

One of the resilient components may exert a force on the armature as it moves into each rest position which prevents the armature from contacting the coil core (or preferably any part of the actuator) that it is moving towards. This may avoid generation of a clicking noise as the armature moves into each rest position which would otherwise be generated by the impact of the armature on a coil core. Each rest position may be an equilibrium position defined by a balance between (a) the force exerted on the armature in the axial direction by the resilient component and (b) the magnetic forces exerted on the armature in the axial direction, which may predominantly be due to attraction of its permanent magnet to the closer of the two coil cores.

The permanent magnet may be polarised in a radial direction relative to the central axis of the actuator. Preferably it has an annular configuration.

In some embodiments, each coil core extends from one end thereof to an opposite end through a respective coil. At least part of the armature may be located around one of the ends of the closer coil core in a plane perpendicular to the central axis of the actuator when the actuator is in each stable rest position. This configuration may serve to reliably hold the armature in each stable rest position due to a strong magnetic attraction between the armature and a radially adjacent portion of the coil core.

Preferably, at least part of the armature is located between the ends of the closer coil core in each stable rest position. This configuration may serve to reliably hold the armature in each stable rest position due to a strong magnetic attraction between the armature and the two ends of the coil core.

In further embodiments, the actuator may include:

an actuator housing; and
a pivotal axis in a fixed position relative to the housing,
wherein the armature is mounted for rotation about the pivotal axis between the first and second stable rest positions.

As the armature of the actuator may swing between positions in which it is located between (but does not come into contact with) the poles of the coil cores, the noise level associated with actuation of the actuator can be reduced.

In each of the first and second positions, the poles of the coil core adjacent to the permanent magnet may be spaced from the permanent magnet in a direction parallel to the pivotal axis of the armature. The actuator may be configured in this way such that the permanent magnet does not contact the poles in each of the first and second positions. This serves to avoid generation of noise due to the armature impacting on the coil core as it moves into each position.

The armature may be held in each of the first and second positions due to interaction of the magnetic flux generated by the permanent magnet and the respective coil core in each position. The armature may be securely held in each position, without electrical energy being consumed by passing a current through either of the coils.

In preferred embodiments, the armature comprises a main body comprising the permanent magnet, a sub-assembly body, and a resilient coupling which resiliently couples the sub-assembly body to the main body. This resilient coupling may comprise the two resilient components referred to above.

The armature's main body and sub-assembly body may be able to pivot relative to each other about the pivotal axis. This configuration provides reduced inertia, faster reaction, reduced power consumption and full magnetic force (torque) available outside the actuator.

The sub-assembly body may be pivotable between first and second end-of-travel positions.

In some embodiments, one of the coils of the actuator has a greater number of turns than the other coil. This may be beneficial in applications where a greater switching force is required when switching in one direction relative to the opposite direction.

The present invention further provides a pinch valve comprising an actuator as described herein, a pinching mechanism which includes or is mechanically coupled to the armature of the actuator; and a supporting base for carrying a resilient tube to be selectively pinched by the pinching mechanism when the armature moves into one of its stable rest positions.

The armature may include a tubing engaging portion (or be mechanically coupled to a component) which defines a profile in a plane perpendicular to the pivotal axis, and the profile may be shaped such that the profile is closer to the base when the armature is in the first position than when the armature is in the second position. Thus, when the armature is in its first position, the tubing engaging portion pinches the tubing between itself and the base. This may substantially close the tubing. When the armature is in the second position, the tubing engaging portion is spaced further from the base such that the tubing is open to allow fluid to flow through it.

By employing an actuator as described herein in a pinch valve, the level of noise generated by operation of the pinch valve may be reduced.

The armature may include a first base engaging portion which engages the supporting base when the armature is in its first position. The first base engaging portion and the base may be configured such that, as the armature swings into its first position, the first base engaging portion is brought into contact with the upper surface of the supporting base at an acute angle, such that they are brought into contact in a smooth and gradual manner. The contact between the first base engaging portion and the supporting base may assist in retaining the armature firmly and reliably in the first position.

The first base engaging portion may comprise a first roller. Provision of a roller having a rotational axis parallel to the pivotal axis of the armature in this manner may further assist in providing a smooth action as the first base engaging portion is brought into contact with the supporting base.

The first roller may define part of the profile of the tubing engaging portion of the armature. This serves to reduce frictional resistance when the armature is brought into contact with the tubing, as the roller is able to roll over the tubing, providing a smoother engagement between them. The roller may comprise a hub with radially extending flanges on each side thereof. The hub may engage the upper surface of the tubing and the flanges may engage the supporting base on either side of the tubing when the armature is in the first position.

Furthermore the armature may include a second base engaging portion which engages the supporting base when the armature is in its second position. The second base engaging portion may comprise a second roller. These features may provide similar benefits to the first roller as discussed above. The characteristics of the actuator may therefore be similar as it moves into the first and second positions, respectively.

In preferred embodiments, the pinch valve includes a removable cartridge, wherein the cartridge includes a length of resilient tubing and the supporting base of the valve which carries the tubing. The length of tubing with which the pinch valve interacts may become worn and degrade over time. Provision of a removable cartridge including the length of tubing facilitates easy replacement of the tubing. This may also ensure that tubing of sufficient quality is used when the tubing is replaced, to reduce the risk of premature failure of the tubing in the valve.

In preferred embodiments, armatures are provided in the form of assemblies that move relative to the coils of the actuator and comprise two parts which are coupled together using one or more resilient couplings. One of these parts comprises a permanent magnet which generates magnetic forces within the actuator. This part is coupled by the resilient coupling(s) to a second part which transfers mechanical force generated by the actuator onto other devices or components. This second part may be a push rod for example or an armature sub-assembly in accordance with preferred embodiments described herein. In these configurations, the part comprising the permanent magnet is able to accelerate relatively quickly. This may mean that a relatively short current pulse is sufficient to be applied to the coils to switch the actuator, thereby reducing energy consumption. The second part then follows the first part in response to forces exerted on the second part by the first part via the resilient coupling(s). In this way, the second part may move more slowly than the first part. Thus, whilst the first part may switch relatively quickly, the second part can switch from one stable rest position to another more slowly. This may be particularly beneficial when using the actuator to exert a force on a viscous fluid. Such a fluid will generate a lower reactive, resistive force if this force is exerted more gradually, with a lower impulse. This leads to more gentle operation, consuming less energy and exerting less stress on components of the actuator.

This two part configuration is also beneficial in that the resilient coupling provides internal energy recycling as energy is stored in the resilient coupling in each stable rest position. This provides rapid acceleration of the first part out of each stable rest position giving a fast actuator response whilst providing reduced energy consumption and providing substantial transfer of magnetic forces generated by the actuator to outside the actuator.

The invention further provides an actuation system including an actuator as described herein and an actuator controller for selectively sending electrical control pulses to the coils of the actuator to switch the armature between the first and second positions.

Furthermore, the present disclosure provides such an actuation system which includes a valve mechanically coupled to the armature of the actuator so that the valve is operable by the actuator. The valve may take various forms. It may be a pinch valve or an inline valve, for example. Preferably, the valve is substantially silent when switching between its open and closed configurations.

The gas supply controller of the gas supply control system is configured to receive a pressure signal from a pressure sensor. The signal may be dependent on or indicative of the gas pressure within a mask worn by a patient, for example. Additionally, the gas supply controller may be configured to receive an oxygen level signal which is dependent on or indicative of the oxygen level in the blood of a patient. For example, the signal may be generated by an oxygen saturation sensor which is responsive to the level of oxygen saturation in a patient's blood. The controller may be arranged to adjust the timing of the switching actions of the actuator between its rest positions in order to open and close the valve, and/or adjust the pressure of the gas being supplied to the valve, having regard to the pressure signal and/or the oxygen level signal, with the aim of maintaining the oxygen level in the patient's blood between upper and lower thresholds.

The present invention also provides a gas supply control system comprising a pinch valve as described herein, a pressure sensor and a gas supply controller, wherein the pressure sensor is configured to generate a pressure signal in response to a gaseous pressure sensed by the sensor, the gas supply controller is communicatively coupled to the pressure sensor so as to receive the pressure signal and also communicatively coupled to the pinch valve, and wherein the gas supply controller is configured to send control signals to the pinch valve in response to the pressure signal, which control signals cause the pinch valve to switch the armature of the pinch valve between its first and second positions. Such a system may be used for example to control a supply of oxygen to a patient.

The pressure sensor may be responsive to the breathing of the patient, and the pressure signal may be employed to control the oxygen supply in time with the patient's breathing cycle.

As the actuator of the valve is able to select either a first or second position and be held in either position without consuming electrical energy, the valve may be switched to be continuously open as a failsafe default mode, in a medical environment for example.

As consumption of electrical energy by the valve actuator is minimised, it may be particularly suitable for portable applications using a battery as a source of power.

It is common for existing oxygen supply systems to provide a continuous flow of oxygen. A large proportion of the oxygen is not inhaled by the patient and is wasted. The present system allows an on-demand metered supply of oxygen thereby reducing the amount of oxygen wastage substantially.

Provision of a pressure sensor for detecting the start of an intake of breath by a patient means that the timing of the dose of oxygen may be provided automatically, without requiring manual adjustment.

The gas supply controller may be configured to generate a switching rate signal dependent on (or providing a value corresponding to) the rate of switching of the valve. This information may be valuable to medical professionals to give an indication of the respiratory rate of the patient. Preferably, the system may include a display to which the signal is outputted to cause the display to show an indication dependent on the switching rate signal.

Brief Description of the Drawings

Embodiments of the invention will now be described by way of example and with reference to the accompanying schematic drawings wherein:

Figures 1 to 3 are perspective cross-sectional views of a linear actuator embodying the present invention with its armature shown in three different positions;
Figure 4 is a cross-sectional side view of another linear actuator embodying the invention in combination with a pinch valve mechanism;
Figures 5 and 6 are perspective views of a pinch valve embodying the present invention with its armature in two different positions;
Figure 7 is an enlarged view of part of Figure 6;
Figures 8 and 9 are perspective views and Figure 10 is a side view of a further pinch valve embodying the invention which includes rollers;
Figures 11, 13 and 14 are side views of a further pinch valve configuration embodying the invention;
Figures 12, 15 and 16 to 18 are perspective views of the pinch valve shown in Figures 11, 13 and 14;
Figure 19 corresponds to Figure 14 and has additional annotations;
Figures 20 and 21 are schematic graphs of torque and pinch forwards respectively, for the pinch valve of Figure 19;
Figure 22 is a diagram illustrating generation of a pinch force using a valve configuration of Figure 19;
Figure 23 is a plan view of magnetic components of a pinch valve embodying the invention;
Figures 24 and 25 are perspective cross-sectional views of a linear actuator embodying the invention in combination with an inline valve, with the armature of the actuator in two different positions;
Figure 26 is a block diagram of a gas supply control system embodying the present invention;
Figure 27 is a block diagram of another gas supply system embodying the present invention; and
Figure 28 is a side view of a patient mask for use in a gas supply control system of the present invention.

Detailed Description of the Drawings

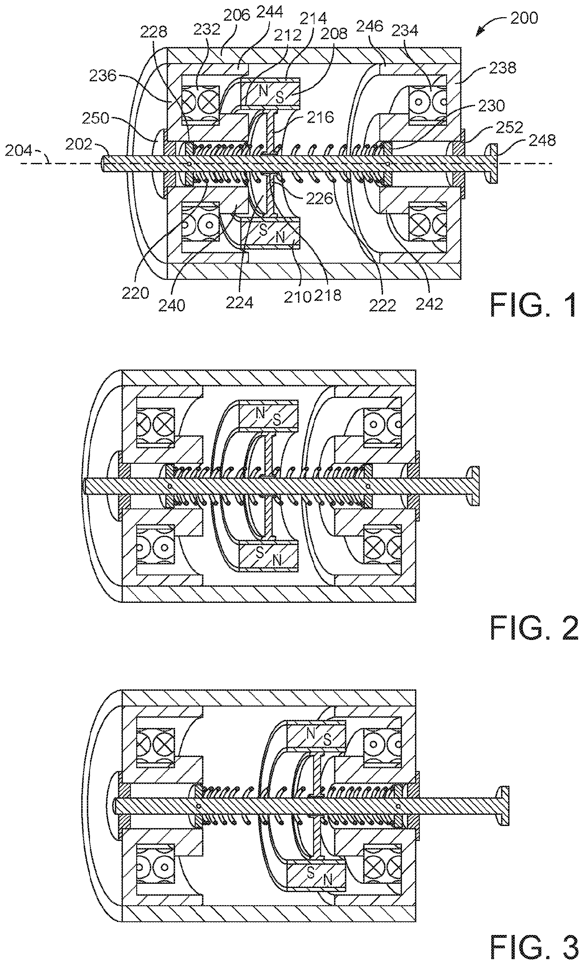

Figures 1 to 3 are cross-sectional views of a compact linear actuator 200 according to an embodiment of the invention. An elongate, linear push rod 202 extends along a central, longitudinal axis 204 of the actuator. The push rod is slidable along this axis relative to a coaxial cylindrical actuator housing 206.

An annular armature 208 extends around the push rod and is coaxially mounted relative to the push rod. The armature comprises a permanent magnet 210 which is provided between radially inner and outer pole pieces 212 and 214. Permanent magnet 210 is polarised in a radial direction.

The armature also includes a magnet mount 216. The magnet mount extends radially inwardly from the inner pole piece 212 towards the push rod 202. The outer circumferential surface of the magnet mount 216 is mounted in a fixed position on an inner circumferential surface of the inner pole piece 212. A circular aperture 218 is defined by a radially inwardly facing surface of the magnet mount 216. The push rod 202 extends through the aperture 218 and is able to move relative to it. In this way, the armature 208 and the push rod 202 are able to slide relative to each other in an axial direction.

A pair of resilient components 220, 222 is provided. In the embodiment illustrated, they are in the form of coil springs, although it will be appreciated that other forms of resilient device may be used. One end of each resilient component bears against a respective one of two opposite, transversely extending side faces 224 and 226 of the magnet mount 216. The other end of each resilient component is mounted in a fixed position on the push rod or retained in a fixed position relative to the push rod. In the embodiment illustrated, the outer ends of the coil springs 220 and 222 are in engagement with respective discs 228 and 230 which are mounted in fixed positions on the push rod 202. Each resilient component is compressed between one of the fixed discs at one end, the magnet mount at its other end.

The actuator includes two wire coils 232 and 234. They are located towards opposite ends of the cylindrical housing 206. A central axis of each coil is coaxial with the central axis 204 of the actuator. The coils are wound in respective opposite directions.

A coil core 236 extends through coil 232 and the coil core 238 extends through coil 234. One end of each coil core extends axially inwardly to form respective annular, inner pole pieces 240 and 242. The opposite end of each coil core extends radially outwardly, past axially outwardly facing surfaces of the respective coils, radially beyond the respective coils, and then axially inwardly to form outer annular pole pieces 244 and 246. In this way, the coil cores form inner and outer pole pieces which are radially spaced apart in a common transverse plane, with a coil core extending between each pair of pole pieces, through the centre of the respective coils.

A handle 248 is provided at one end of the push rod 202. In the embodiment illustrated, this is in the form of a disc which is integrally formed with the push rod. This facilitates manual switching of the actuator if required.

Each coil core 236 and 238 is fixedly mounted on a radially inwardly facing surface of the housing 206, at axially opposite ends of the housing. A pair of seals 250 and 252 extend radially inwardly between a respective coil core and the push rod 202 at opposite ends of the push rod to close each end of the housing around the push rods. Each coil core forms a respective radially extending end face of the actuator housing.

The linear actuator of Figures 1 to 3 has two stable rest positions. A first stable rest position is shown in Figure 1. In the configuration of Figure 1, the armature 208 is located closer to the coil core 236. Also, the push rod 202 is located in its left-most position. A second stable rest position is shown in Figure 3, in which the armature is located closer to the coil core 238 and the push rod is in its right-most position relative to the housing. In each rest position, the armature is attracted towards the adjacent coil core by magnetic forces generated by the permanent magnet 210. Magnetic flux generated by the magnet links with the coil core to form a magnetic circuit. This magnetic force urges the armature toward the adjacent coil core. In each stable rest position, this force is balanced by an opposing mechanical force generated by one of the resilient components 220 and 222. In each rest position, one of the resilient components may be compressed to a greater extent than the other, or one may be compressed and the other substantially uncompressed.

As the armature moves into each rest position, one of the resilient components will be compressed when the armature moves relative to the push rod, when further movement of the push rod is constrained externally or due to one of the discs 228 or 230 contacting an interior surface of the actuator.

In each rest position, the armature is spaced from the adjacent coil core by a gap. It does not therefore come into contact with the adjacent coil core as it moves into each stable rest position. This avoids generation of a clicking noise as the armature moves into each stable rest position.

The armature may be separated from the adjacent coil core by a fluid filled gap. The fluid may be a gas such as air.

The inner and outer poles of each coil core may be spaced apart in the radial direction such that the distance between them is greater than the radial extent of the annular armature formed by permanent magnet 210 and pole pieces 212 and 214. In this case, in each stable rest position, the armature may overlap with one or both of the poles of the coil core to some extent in the axial direction.

Figure 2 shows the actuator in an unstable, neutral configuration, with the armature located mid-way between the coil cores. In practice, the armature will not rest in this position, but move through it when switching from one stable rest position to the other.

The armature may be switched from one stable rest position to another by briefly energising one or both of the coils.

For example, the actuator may be switched from the configuration of Figure 1 to that of Figure 3 by applying a brief current pulse to the coil 232. This pulse should be sufficient to reduce the magnetic attraction between the armature and the coil core to such an extent that resilient component 220 pushes the armature away from the starting rest position, towards the stable rest position of Figure 3. The pulse should also be sufficient to ensure that the armature is reliably attracted into the other stable rest position by magnetic forces acting between the permanent magnet and the opposite coil core 238. Application of a suitable pulse to the coil 234 will result in the armature being switched back in a similar manner to its original stable, rest position as shown in Figure 1.

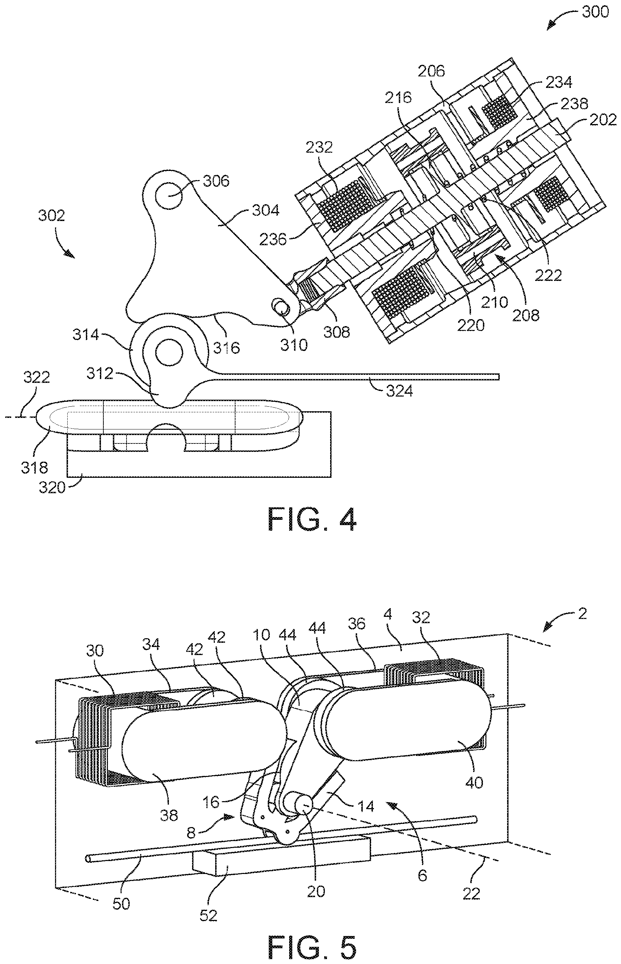

An alternative linear actuator configuration is shown in Figure 4, in combination with a pinch valve mechanism. The same reference numerals are used to denote the same or similar features of this actuator 300 to those of the actuator 200 shown in Figures 1 to 3.

In this embodiment, the armature 208 is mounted in a fixed position on the push rod 202. The outer ends of the resilient components 220 and 222 bear against respective coil cores 236 and 238. Coil 232 is shown as having a greater number of windings than coil 234. This is so that the actuator is able to provide a greater force when switching in one direction than the other (that is, to close the valve in the embodiment illustrated). The operation of the actuator 300 is similar to that of actuator 200.

One end of the push rod 202 is coupled to a pinching mechanism 302. The mechanism includes a cam member 304 which is mounted for rotation about a pivot 306 of the pinching mechanism. A coupling 308 is pivotally mounted on the cam member 304 for rotation relative to the cam member about a second pivot 310. The coupling 308 is connected to one end of the push rod 202.

The pinching mechanism 302 includes a pinching element 312 which has a wheel 314 rotatably mounted thereon. The wheel 314 acts as a cam follower, following a cam surface 316 formed on the cam member 304. The pinching element 312 is in engagement with a tube 318 carried by a support member 320. The cam member is shaped so as to convert linear movement of the push rod 202 caused by actuator 300 into movement of the pinching element 312 in a direction perpendicular to the longitudinal axis 322 of the tube 318. In some cases, it may be preferable for the pinching element to move in a direction perpendicular to the tube, rather than the motion of the pinching element having a component in an axial direction, to minimise wear on the tube and axial pulling forces on the tube.

In Figure 4, actuator 300 is shown in its neutral, unstable condition, with the armature located midway between the coil cores 236 and 238.

The pinching element 312 is biased upwardly towards the cam member by an arm 324 to keep the cam follower wheel 314 in contact with cam surface 316. In one stable rest position of the actuator, the tube 318 is open. When the actuator moves into its other stable rest position, the pinching element is moved downwardly, towards the tube, pinching the tube between the pinching element and the support member 320, so as to close the tube.

Another pinch valve embodying the invention will now be described with reference to Figures 5 to 7. The valve 2 is provided within a housing 4. The housing is only shown partly in Figure 5 for the purposes of clarity in the drawings. The valve includes an actuator 6.

The actuator has an armature 8. The armature has an armature body 10 which includes a permanent magnet 12. The armature also includes a sub-assembly body 14. A resilient coupling in the form of a pair of leaf springs 16 resiliently couples the sub-assembly body to the armature body. It will be appreciated that this resilient coupling may be implemented in other ways. A torsion bar or torsion spring may be used for example.

A pivot 20 is mounted in a fixed position on the housing 4. A pivotal axis 22 extends axially through the centre of the pivot 20. The armature body and the sub-assembly body are each mounted on the pivot for rotation about the pivotal axis.

The actuator includes two coils 30 and 32. Each coil is wound around a respective coil core 34 and 36. Each coil core comprises a pair of arms 38 and 40 on either side of the coil. The arms extend transversely with respect to the pivotal axis. A pair of poles 42, 44 is provided at the distal ends of each pair of arms.

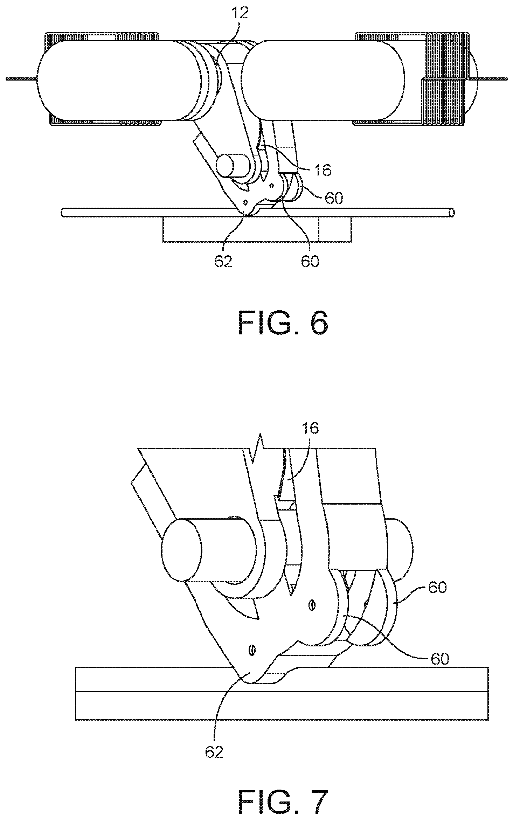

In the configuration shown in Figure 5, the permanent magnet of the armature is located between the poles 44. This represents a stable rest position of the armature. Figure 6 shows a second stable rest position, in which the permanent magnet 12 is located between the other pair of poles 42.

The valve includes a length of resilient tubing 50 which is carried by a supporting base 52.

A pair of rounded flanges 60 extend in a direction which is transverse with respect to the pivotal axis, and away from the sub-assembly body. In the configuration shown in Figure 5, it can be seen that the flanges 60 are in contact with the supporting base 52 on either side of the tubing 50. In this position, the tubing is not compressed by the sub-assembly body and fluid is able to flow freely through the tubing.

In the other armature position shown in Figures 6 and 7, a tubing engaging portion 62 of the sub-assembly body is pinching the tubing located between it and the supporting base 52 to substantially close the tubing. The tubing engaging portion 62 provides a convex surface curved in a plane perpendicular to the pivotal axis of the actuator to enable it to be brought smoothly into engagement with the tubing, with a view to reducing wear of the tubing.

The permanent magnet is orientated with its poles aligned in a direction parallel to the pivotal axis. In each of its rest positions, magnetic flux generated by the magnet links with the adjacent coil core so that the armature is retained in a stable rest position by magnetic forces generated by the magnet.

In order to switch the armature from one stable rest position to the other, the coil associated with the current rest position is energised to repel the magnet, and the magnet pulls itself towards the other coil core. In this way, the pinch valve can be switched to open or close the tubing 50 using a single coil driving technique.

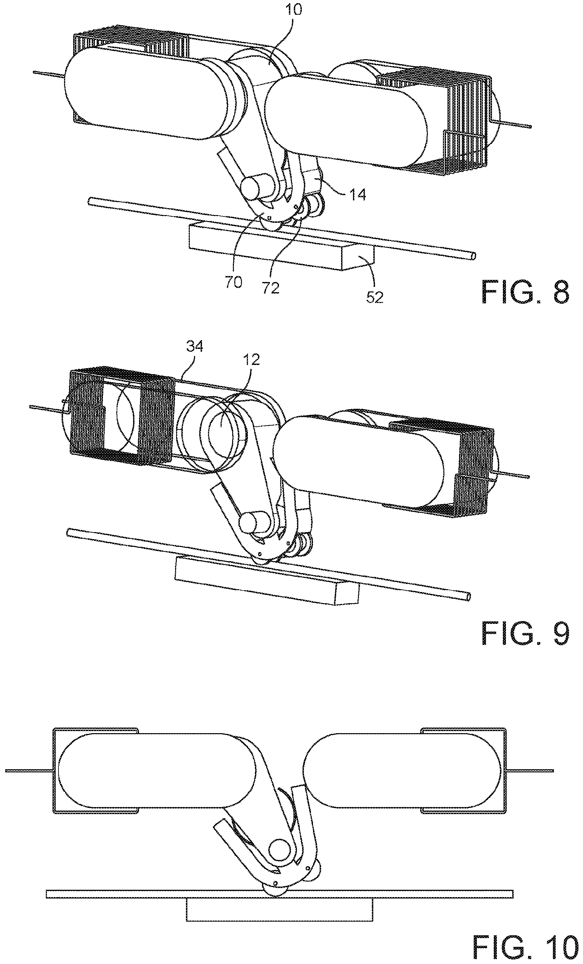

Figures 8 to 10 show another pinch valve embodying the invention which is similar to that of Figures 5 to 7. A first roller 70 is provided instead of the tubing engaging portion 62, and a second roller 72 is provided instead of the flanges 60, as part of the sub-assembly 14. Each roller comprises a pair of flanges which are brought into contact with the supporting base 52 as the armature moves into a respective stable end position. The diameter of the hub of the second roller 72 which extends between its flanges is dimensioned such that it does not compress the tubing. The first roller 70 is dimensioned so as to pinch the tubing to the desired extent as it is brought into contact with it.

In Figure 9, the coil core 34 is shown in outline only so that the permanent magnet 12 is more clearly visible.

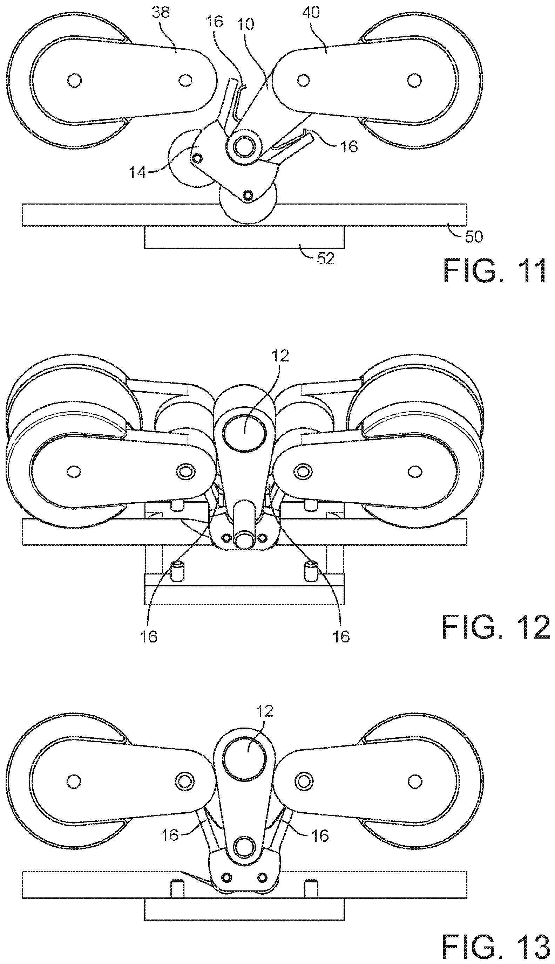

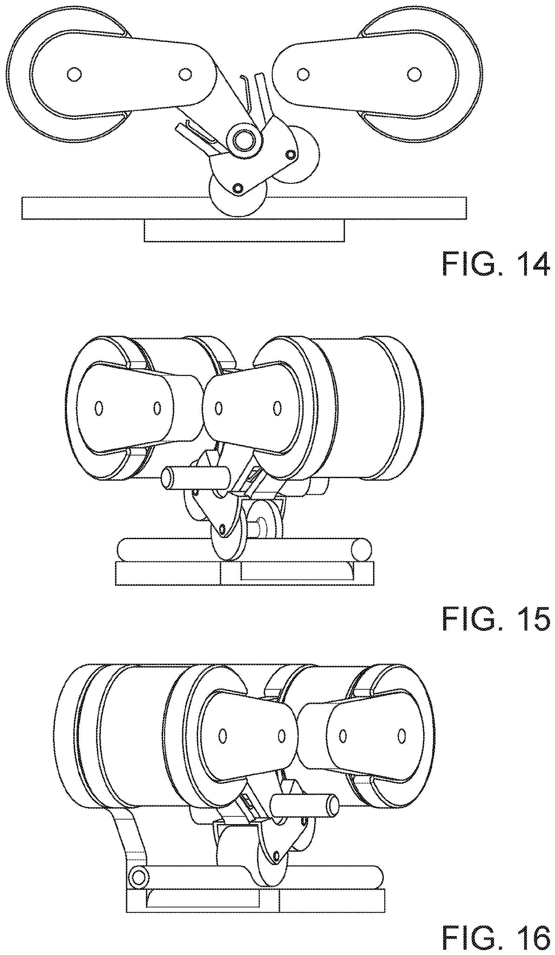

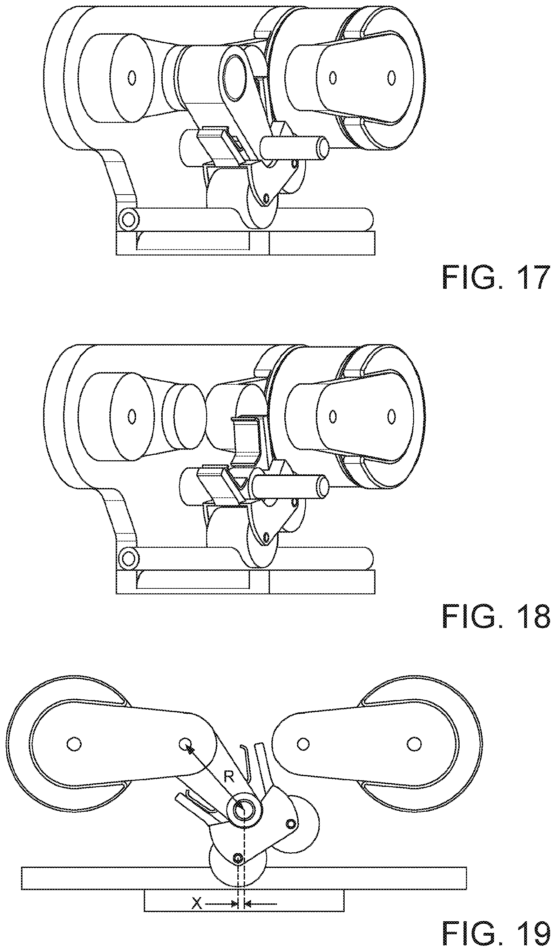

Figures 11 to 18 show side and perspective views of a further pinch valve embodying the invention which is similar to that shown in Figures 8 to 10. In Figure 17, one of the coils and part of its core are omitted so that the armature configuration is more easily visible. In Figure 18, the armature body is omitted so that the sub-assembly and resilient couplings are more readily visible.

Figures 12 and 13 show an intermediate configuration in which the armature body 10 is in a neutral, unstable position, midway between the two stable rest positions. In this position, the two resilient couplings, in this case in the form of leaf springs 16, are both uncompressed, or slightly compressed, each to the same extent.

In Figure 19, the distance "R" is marked which corresponds to the distance between the centre of one pair of pole pieces and the pivotal axis. A distance "X" is marked which is the distance that the centre of the first roller is offset from a perpendicular line extending through the pivotal axis from the supporting base. The ratio R/X is equal to the leverage ratio for this configuration. Preferably, R is several times greater than X in order to provide a substantial pinching force.

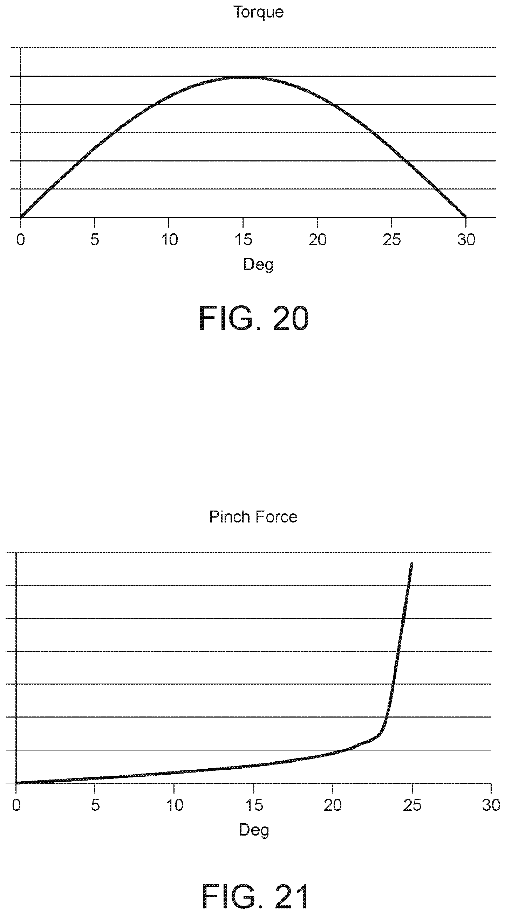

Figure 20 is a schematic plot of the torque exerted on the armature against angle. In this graph, 0 and 30° correspond to the central position and one end of travel of the armature, respectively. It represents the torque attributable to the magnetic forces generated by the permanent magnet alone, without the coils being energised.

Figure 21 is a plot of pinch force against armature position by way of example. A substantial pinch force may be generated in a valve embodying the present invention to reliably close tubing compressed by the pinch valve.

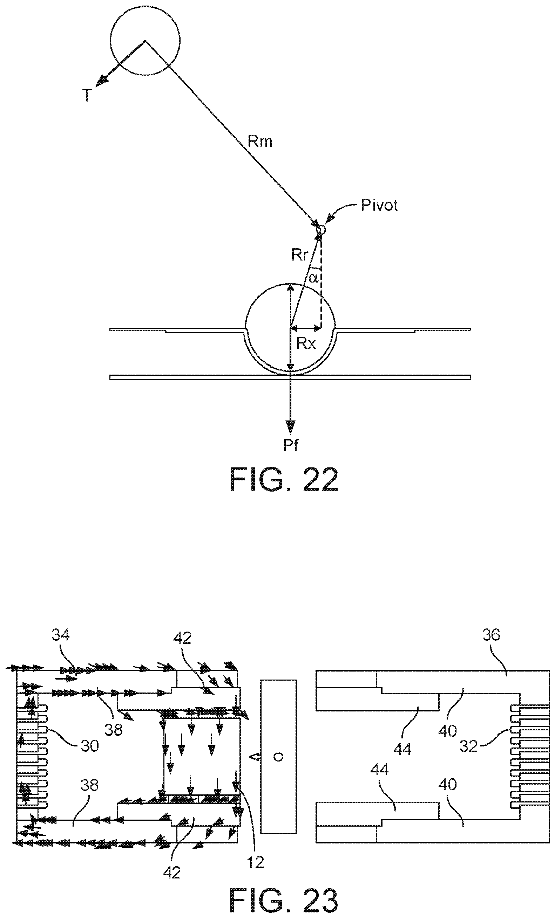

This arrangement depicted in Figure 19 is shown diagrammatically in Figure 22. With reference to the dimensions labelled in Figure 22:

$$Pf = T*Rm/Rr*\sin(\alpha)$$

where:

Pf = pinching force;
T = torque acting on the armature;
Rm = the distance between the centre of the permanent magnet and the pivotal axis of the armature;
Rr = the distance between the centre of the pinching roller and the pivotal axis; and
$\alpha$ = the angle subtended between a line between the pinching roller axis and the pivotal axis of the armature and a perpendicular line through the pivotal axis from the supporting base.

It can be seen that:

$$Pf = T*Rm/Rr*(Rx/Rr)$$
$$= T*Rm/Rx$$

Figure 23 is a diagram showing a plan view of an actuator embodying the invention. The permanent magnet 12 is located between the poles 42 of the coil core associated with the coil 30. The rectangle in the centre of the diagram represents the pivot of the armature.

A plurality of black arrows have been superimposed on the diagram to represent magnetic flux generated by the permanent magnet. The size of each arrow is proportional to the magnetic field strength at its location and the direction of the arrow shows the direction of the magnetic flux at its location. It can be seen that the magnetic flux generated by the magnet acts to securely retain the magnet between the adjacent poles.

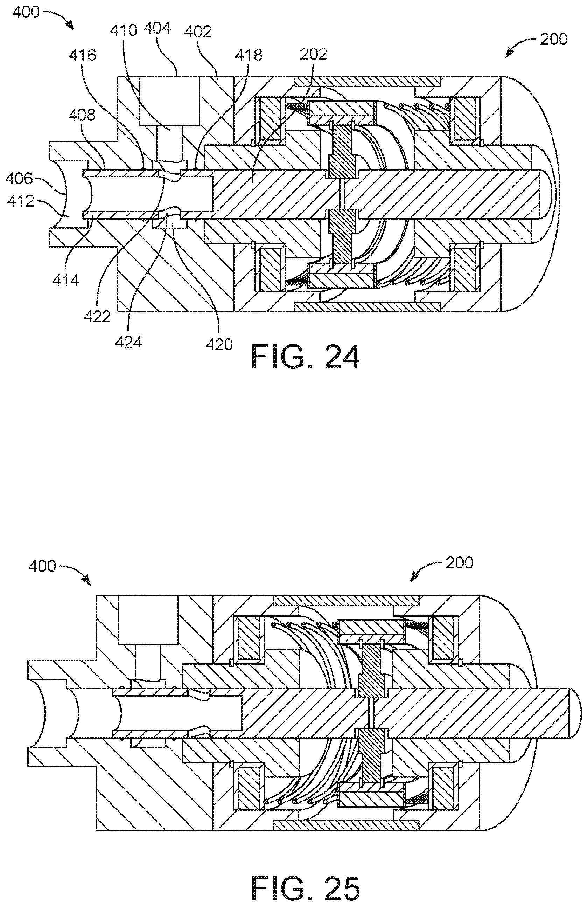

Figures 24 and 25 show a linear actuator 200 similar to that depicted in Figures 1 to 3 in combination with an inline valve 400. In some applications, it may be preferable to use an inline valve rather than a pinch valve.

The valve body 402 is mounted onto one end of the housing of the actuator 200. The body 402 defines a fluid inlet port 404 and a fluid outlet port 406. The inlet and outlet ports are coupled via inlet and outlet conduits 410 and 412 to a cylindrical bore 408 which is also defined by the valve body. A sleeve 414 is provided which is a close sliding fit within the bore 408. A fluid-tight seal is provided between the outer surface of the sleeve and the bore on each side of the inlet 410 by seals 416 and 418.

The diameter of the bore 408 is increased around the location where the inlet meets the bore to form a chamber 420 which extends around the outside of the sleeve 414. An opening or a pair of openings 422 and 424 is/are defined by the wall of the sleeve which extend radially through the sleeve. The sleeve is attached to one end of the push rod 202 of the actuator or integrally formed with the push rod. Movement of the push rod by the actuator therefore moves the sleeve along bore 408 of the valve.

In one stable rest position of the actuator as shown in Figure 24, the openings coincide with the chamber 420. Thus, in this valve configuration, the valve is open as its inlet port is in fluid communication with its outlet port via the inlet conduit 410, chamber 420, openings 422, 424, the interior of sleeve 414 and the outlet conduit 412.

In the other stable rest position of the actuator as shown in Figure 25, the sleeve has been axially displaced by the actuator within bore 408, so that the openings 422, 424 are no longer within chamber 420. The valve is then closed as the sleeve has closed the fluid path between the inlet and outlet ports.

The axial extent of the chamber 420 is preferably greater than that of the opening(s) in the wall of the sleeve. This allows a greater degree of tolerance in the axial positions of the sleeve that will allow fluid to flow through the valve. This may be advantageous as the stable rest positions of the actuator at each end of the travel of its push rod are dependent on spring forces, rather than contact between rigid moving and stationary parts of the actuator. As a result, there may be some variation in the location of the push rod when the actuator has been switched to open the valve which is accommodated by the relative axial dimensions of the chamber and the opening(s).

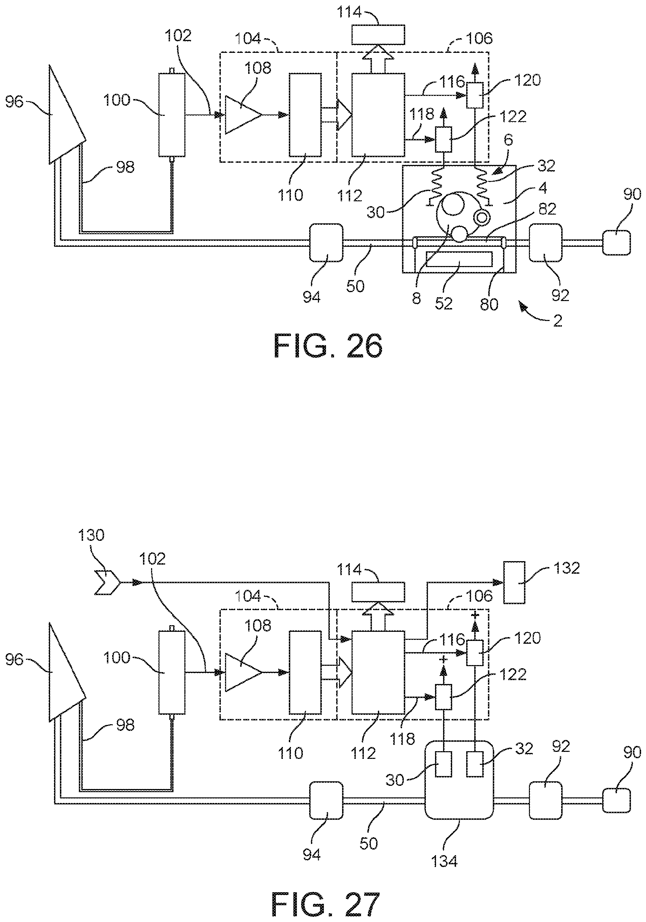

Figure 26 is a block diagram representing a gas supply control system incorporating a pinch valve embodying the present invention, in a preferred use as an Intelligent Medical Oxygen System (IMOS). The pinch valve 2 is represented schematically in the drawing and is arranged to control the flow of gas through tubing 50.

The pinch valve includes a cartridge. In preferred configurations the cartridge is removable. The cartridge includes a length of resilient tubing 82 and the supporting base 52 of the pinch valve. The cartridge may be removed from the pinch valve and replaced with a new cartridge as and when required due to wear of the tubing 82 by the valve. This modular cartridge construction ensures that the replacement of the tubing can be achieved reliably, without requiring a high degree of skill or training.

A connector 90 is provided to connect the tubing 50 to a supply of oxygen. This supply is in turn fed through an oxygen pressure regulator 92 before being fed through the pinch valve 2.

A flow indicator 94 monitors the flow rate of the gas passed through the pinch valve along tubing 50.

The supply of oxygen is then fed to a mask 96 for use by a patient. A pressure sensor tubing 98 is coupled to the interior of the mask at one end and to a pressure sensor 100 at the other.

The pressure sensor 100 is configured to generate an electrical pressure signal along output 102. This signal is communicatively coupled to a gas supply controller 104 which includes an actuator controller 106.

The pressure signal is received by a preamplifier 108, the output of which is coupled to an analogue-to-digital converter 110. The output from converter 110 is inputted into a microcontroller 112 of the actuator controller. A display 114 is communicatively coupled to the microcontroller 112. The microcontroller outputs control signals on lines 116 and 118 which are coupled to drivers 120 and 122 for respective coils 32 and 30 of the actuator.

The pressure sensor 100 is able to detect a pressure drop within the mask 96 indicative of a patient beginning an intake of breath. The microcontroller may respond to signals derived from the output of the pressure sensor to open and close the pinch valve as appropriate to feed an appropriate supply of oxygen to the mask along tubing 50 in time with the breathing cycle of the patient. Alternatively, or in addition, the microcontroller may respond to signals derived from the output of the pressure sensor by adjusting the oxygen pressure regulator 92 so as to alter the pressure of the oxygen supplied to the valve 2.

Figure 27 is a further block diagram of a gas supply control system embodying the invention. It illustrates a number of possible modifications that may independently be made to a system exemplified by the configuration shown in Figure 26. The same reference numerals are used to identify the same or similar features in Figure 27.

A gas supply control system embodying the invention may optionally include an oxygen saturation sensor 130. As shown in Figure 27, such a sensor may be communicatively coupled to the microcontroller 112. The microcontroller may be configured to adjust the control signals sent to the actuator having regard to signals received from the sensor 130 with a view to maintaining the level of oxygen in a patient's blood within predetermined thresholds. Alternatively, or additionally, the microcontroller may adjust the pressure of the gas being supplied to the valve by adjusting the oxygen pressure regulator 92 having regard to signals received from the sensor 130.

The microcontroller 112 may be communicatively coupled to a transmitter 132. This allows the system to transmit data wirelessly to a receiver on another device or a network (via a Bluetooth link for example) so that information regarding the current and/or historical operation of the system can be monitored and/or recorded for subsequent use.

Instead of the pinch valve assembly shown in Figure 26, an alternative valve assembly 134 may be used. For example, the valve assembly may consist of an actuator as described herein and an inline valve. This combination may be of the form shown in Figures 24 and 25 for example.

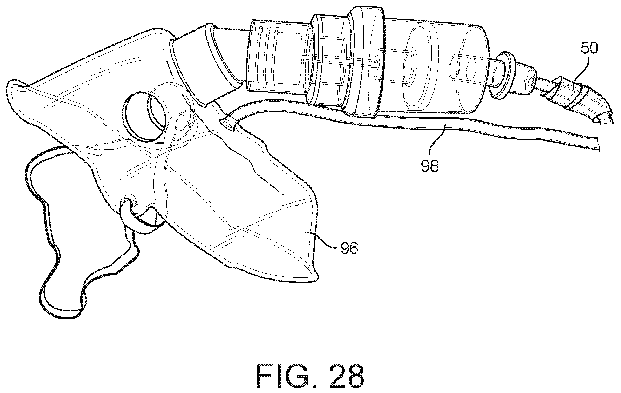

Figure 28 is a side view of a mask which is adapted for use in the systems of Figures 26 and 27. In addition to an inflow tubing 50, it also includes a pressure sensor tubing 98 which is coupled to the interior space defined by the mask.

## Claims

1. A gas supply control system comprising:

   an actuation system including an electromagnetic actuator (6, 200, 300) comprising an armature (8, 208) comprising a permanent magnet (12, 208), two coils (30, 32; 232, 234), and two coil cores (34, 36; 236, 238), each of which extends within a respective coil, wherein the armature is moveable relative to the coils between first and second stable rest positions by passing a current through at least one of the coils, and in each rest position, the armature is closer to one of the coil cores than the other of the coil cores and is spaced by a gap from the closer coil core;
   a gas supply controller (104) for selectively sending electrical control pulses to the coils of the actuator to switch the armature between the first and second positions; and
   a valve (2, 134) mechanically coupled to the armature of the actuator so that the valve is operable by the actuator,
   wherein the gas supply controller (104) is configured to receive a pressure signal from a pressure sensor (100).

2. A system of claim 1, wherein the actuator includes two resilient components (16, 220, 222), wherein one of the resilient components is compressed by movement of the armature towards each stable rest position, and preferably the actuator further includes a push rod (202) which extends along a central axis of the actuator and is moveable relative to the coils along the central axis (204), wherein the armature is axially slideable along the push rod, wherein preferably one end of each resilient component is mounted in a fixed position on the push rod and the armature exerts a compressive force against the other end of one of the resilient components by sliding along the push rod as it moves towards each stable rest position.

3. A system of claim 2, wherein one of the resilient components exerts a force on the armature as it moves into each rest position which prevents the armature from contacting the coil core that it is moving towards.

4. A system of claim 2 or claim 3, wherein the permanent magnet (208) is polarised in a radial direction relative to the central axis (204) of the actuator.

5. A system of any preceding claim, wherein each coil core (34, 36; 236, 238) extends through a respective coil between ends of the coil core, at least part of the armature (8, 208) is located between the ends of the closer coil core in each stable rest position.

6. A system of claim 1 or claim 2, wherein the actuator includes:

an actuator housing (4); and
a pivotal axis (22) in a fixed position relative to the housing,
wherein the armature (8) is mounted for rotation about the pivotal axis between the first and second stable rest positions.

7. A system of claim 6, wherein the armature (8) comprises a main body (10) comprising the permanent magnet (12), a sub-assembly body (14), and a resilient coupling (16) which resiliently couples the sub-assembly body to the main body, and preferably the sub-assembly body is pivotable between first and second end-of-travel positions.

8. A system of any preceding claim, wherein the valve is a pinch valve comprising:

a pinching mechanism (302) which is provided by or mechanically coupled to the armature of the actuator; and
a supporting base (52, 320) for carrying a resilient tube to be selectively pinched by the pinching mechanism when the armature moves into one of its stable rest positions.

9. A system of claim 8, when dependent directly or indirectly on claim 6 or claim 7, wherein the armature includes a tubing engaging portion (62) which defines a profile in a plane perpendicular to the pivotal axis, and the profile is shaped such that the profile is closer to the base when the armature is in the first position than when the armature is in the second position, the armature preferably includes a first base engaging portion which engages the supporting base when the armature is in its first position, the first base engaging portion preferably comprises a first roller (70), the first roller preferably defines part of the profile of the tubing engaging portion of the armature, the armature preferably includes a second base engaging portion (60) which engages the supporting base when the armature is in its second position, and the second base engaging portion preferably comprises a second roller (72).

10. A system of claim 8 or claim 9 including a removable cartridge, wherein the cartridge includes a length of resilient tubing (82) and the supporting base (52) of the valve which carries the tubing.

11. A system of any of claims 1 to 7, wherein the valve is an inline valve (400).

12. A system of any preceding claim including pressure sensor tubing (98) for coupling to a mask (96) of a medical oxygen system at one end and coupled to the pressure sensor (100) at the other end.

13. A system of any preceding claim, wherein the gas supply controller (104) is configured to receive an oxygen level signal from an oxygen saturation sensor (130).

14. A system of claim 13, wherein the gas supply controller (104) is configured to generate control signals to adjust the pressure of a gas supplied to the valve.

15. A system of claim 13 or claim 14, wherein the system includes a display (114) and the gas supply controller (104) is configured to generate a switching rate signal dependent on the rate of switching of the valve and output the signal to the display which causes the display to display an indication dependent on the switching rate signal.

**Patentansprüche**

1. Gaszufuhrsteuersystem, umfassend:

ein Betätigungssystem mit einem elektromagnetischen Betätigungsglied (6, 200, 300) umfassend einen Anker (8, 208) umfassend einen Dauermagneten (12, 208), zwei Spulen (30, 32; 232, 234) und zwei Spulenkerne (34, 36; 236, 238), von denen sich jeder in einer jeweiligen Spule erstreckt, wobei der Anker in Relation zu den Spulen zwischen ersten und zweiten stabilen Ruhepositionen durch Leiten eines Stroms durch mindestens eine der Spulen bewegbar ist, und wobei in jeder Ruheposition der Anker näher zu einem der Spulenkerne als dem anderen der Spulenkerne ist und durch einen Spalt von dem näher gelegenen Spulenkern beabstandet ist;

eine Gaszufuhrsteuerung (104) zum gezielten Senden elektrischer Steuerimpulse an die Spulen des Betätigungsglieds, um den Anker zwischen den ersten und zweiten Positionen umzuschalten; und

ein Ventil (2, 134), das mit dem Anker des Betätigungsglieds derart mechanisch gekoppelt ist, dass das Ventil durch das Betätigungsglied betreibbar ist,

wobei die Gaszufuhrsteuerung (104) ausgelegt ist, ein Drucksignal von einem Drucksensor (100) zu erhalten.

2. System nach Anspruch 1, wobei das Betätigungsglied zwei elastische Komponenten (16, 220, 222) umfasst, wobei eine der elastischen Komponenten durch eine Bewegung des Ankers hin zu jeder stabilen Ruheposition zusammengedrückt wird, und wobei vorzugsweise der Anker ferner eine Schubstange (202) umfasst, die sich entlang einer Mittelachse des Betätigungsglieds erstreckt und in Relation zu den Spulen entlang der Mittelachse (204) bewegbar ist, wobei der Anker entlang der Schubstange axial verschiebbar ist, wobei vorzugsweise ein Ende jeder elastischen Komponente in einer festen Position an der Schubstange angebracht ist und der Anker eine Druckkraft auf das andere Ende einer der elastischen Komponenten durch Verschieben entlang der Schubstange aufbringt, während er sich hin zu jeder stabilen Ruheposition bewegt.

3. System nach Anspruch 2, wobei eine der elastischen Komponenten eine Kraft auf den Anker aufbringt, während er sich in jede Ruheposition bewegt, was verhindert, dass der Anker mit dem Spulenkern, zu dem er sich hin bewegt, in Kontakt kommt.

4. System nach Anspruch 2 oder Anspruch 3, wobei der Dauermagnet (208) in einer radialen Richtung in Relation zu der Mittelachse (204) des Betätigungsglieds polarisiert ist.

5. System nach einem der vorhergehenden Ansprüche, wobei sich jeder Spulenkern (34, 36; 236, 238) durch eine jeweilige Spule zwischen Enden des Spulenkerns erstreckt, wobei sich in jeder stabilen Ruheposition zumindest ein Teil des Ankers (8, 208) zwischen den Enden des näher gelegenen Spulenkerns befindet.

6. System nach Anspruch 1 oder Anspruch 2, wobei das Betätigungsglied umfasst:

ein Betätigungsgliedgehäuse (4); und
eine Schwenkachse (22) in einer festen Position in Relation zu dem Gehäuse,
wobei der Anker (8) für eine Drehung um die Schwenkachse zwischen den ersten und zweiten stabilen Ruhepositionen angebracht ist.

7. System nach Anspruch 6, wobei der Anker (8) einen Hauptkörper (10), der den Dauermagneten (12) umfasst, und einen Unterbaugruppenkörper (14) und eine elastische Kopplung (16) umfasst, die den Unterbaugruppenkörper mit dem Hauptkörper elastisch koppelt, und wobei vorzugsweise der Unterbaugruppenkörper zwischen ersten und zweiten Verfahrendpositionen schwenkbar ist.

8. System nach einem der vorhergehenden Ansprüche, wobei das Ventil ein Quetschventil ist, das umfasst:

einen Quetschmechanismus (302), der durch den Anker des Betätigungsglieds bereitgestellt oder mechanisch damit gekoppelt ist; und
eine tragende Basis (52, 320) zum Aufnehmen eines elastischen Schlauchs, der durch den Quetschmechanismus gezielt gequetscht wird, wenn sich der Anker in eine seiner stabilen Ruhepositionen bewegt.

9. System nach Anspruch 8 wenn direkt oder indirekt abhängig von Anspruch 6 oder Anspruch 7, wobei der Anker einen Schlaucheingriffsabschnitt (62) umfasst, der in einer Ebene senkrecht zu der Schwenkachse ein Profil definiert, und wobei das Profil derart geformt ist, dass das Profil näher an der Basis ist, wenn sich der Anker in der ersten Position befindet, als wenn sich der Anker in der zweiten Position befindet, wobei der Anker vorzugsweise einen ersten Basiseingriffsabschnitt umfasst, der die tragende Basis in Eingriff nimmt, wenn sich der Anker in seiner ersten Position befindet, wobei der erste Basiseingriffsabschnitt vorzugsweise eine erste Rolle (70) umfasst, wobei die erste Rolle vorzugsweise einen Teil des Profils des Schlaucheingriffsabschnitts des Ankers definiert, wobei der Anker vorzugsweise einen zweiten Basiseingriffsabschnitt (60) umfasst, der die tragende Basis in Eingriff nimmt, wenn sich der Anker in seiner zweiten Position befindet, und wobei der zweite Basiseingriffsabschnitt vorzugsweise eine zweite Rolle (72) umfasst.

10. System nach Anspruch 8 oder Anspruch 9, umfassend eine entfernbare Kartusche, wobei die Kartusche eine Länge an elastischen Schläuchen (82) und die tragende Basis (52) des Ventils umfasst, die die Schläuche aufnimmt.

11. System nach einem der Ansprüche 1 bis 7, wobei das Ventil ein Inline-Ventil (400) ist.

12. System nach einem der vorhergehenden Ansprüche, umfassend Drucksensorschläuche (98) zum Koppeln mit einer Maske (96) eines medizinischen Sauerstoffsystems an einem Ende und gekoppelt mit dem Drucksensor (100) an dem anderen Ende.

13. System nach einem der vorhergehenden Ansprüche, wobei die Gaszufuhrsteuerung (104) ausgelegt ist, ein Sauerstoffgehaltsignal von einem Sauerstoffsättigungssensor (130) zu erhalten.

14. System nach Anspruch 13, wobei die Gaszufuhrsteuerung (104) ausgelegt ist, Steuersignale zum Anpassen des Drucks eines dem Ventil zugeführten Gases zu erzeugen.

15. System nach Anspruch 13 oder Anspruch 14, wobei das System eine Anzeige (114) umfasst und die Gaszufuhrsteuerung (104) ausgelegt ist, ein Schalthäufigkeitssignal in Abhängigkeit von der Häufigkeit eines Schaltens des Ventils zu erzeugen und das Signal an die Anzeige auszugeben, was die Anzeige veranlasst, einen Hinweis in Abhängigkeit von dem Schalthäufigkeitssignal anzuzeigen.

**Revendications**

1. Système de régulation d'alimentation en gaz, comprenant :

un système d'actionnement comportant un actionneur électromagnétique (6, 200, 300) comprenant une armature (8, 208) comprenant un aimant permanent (12, 208), deux bobines (30, 32 ; 232, 234) et deux noyaux de bobine (34, 36 ; 236, 238), dont chacun s'étend à l'intérieur d'une bobine respective, l'armature étant mobile par rapport aux bobines entre des première et deuxième positions de repos stables par passage d'un courant à travers au moins une des bobines et, dans chaque position de repos, l'armature étant plus proche d'un des noyaux de bobine que de l'autre des noyaux de bobine et étant espacée du noyau de bobine plus proche par un intervalle ;
un régulateur d'alimentation en gaz (104) destiné à envoyer sélectivement des impulsions électriques de commande aux bobines de l'actionneur afin de commuter l'armature entre les première et deuxième positions ; et
une valve (2, 134) accouplée mécaniquement à l'armature de l'actionneur de façon à ce que la valve soit manœuvrable par l'actionneur,
le régulateur d'alimentation en gaz (104) étant configuré pour recevoir un signal de pression depuis un capteur de pression (100).

2. Système selon la revendication 1, dans lequel l'actionneur comporte deux composants élastiques (16, 220, 222), un des composants élastiques étant comprimé par déplacement de l'armature vers chaque position de repos stable, et, de préférence, l'actionneur comportant en outre une tige-poussoir (202) qui s'étend suivant un axe central de l'actionneur et est mobile par rapport aux bobines suivant l'axe central (204), l'armature étant apte à coulisser axialement le long de la tige-poussoir, de préférence une extrémité de chaque composant élastique étant montée dans une position fixe sur la tige-poussoir et l'armature exerçant une force de compression contre l'autre extrémité d'un des composants élastiques par coulissement le long de la tige-poussoir tandis qu'elle se déplace vers chaque position de repos stable.

3. Système selon la revendication 2, dans lequel un des composants élastiques exerce une force sur l'armature tandis qu'elle adopte chaque position de repos pour ainsi empêcher l'armature de venir au contact du noyau de bobine vers lequel elle se déplace.

4. Système selon la revendication 2 ou la revendication 3, dans lequel l'aimant permanent (208) est polarisé dans une direction radiale par rapport à l'axe central (204) de l'actionneur.

5. Système selon l'une quelconque des revendications précédentes, dans lequel chaque noyau de bobine (34, 36 ; 236, 238) s'étend au travers d'une bobine respective entre des extrémités du noyau de bobine, au moins une partie de l'armature (8, 208) est située entre les extrémités du noyau de bobine plus proche dans chaque position de repos stable.

6. Système selon la revendication 1 ou la revendication 2, dans lequel l'actionneur comporte :

un boîtier (4) d'actionneur ; et
un axe de pivotement (22) dans une position fixe par rapport au boîtier,
l'armature (8) étant montée à rotation autour de l'axe de pivotement entre les première et deuxième positions de repos stables.

7. Système selon la revendication 6, dans lequel l'armature (8) comprend un corps principal (10) comprenant l'aimant permanent (12), un corps sous-ensemble (14) et un accouplement élastique (16) qui accouple élastiquement le corps sous-ensemble au corps principal et, de préférence, le corps sous-ensemble est apte à pivoter entre des première et deuxième positions de fin de course.

8. Système selon l'une quelconque des revendications précédentes, dans lequel la valve est une valve à pincement comprenant :

un mécanisme pinceur (302) qui est fourni par l'armature de l'actionneur ou accouplé mécaniquement à celle-ci ; et

un socle support (52, 320) destiné à porter un tube élastique devant être pincé sélectivement par le mécanisme pinceur lorsque l'armature adopte une de ses positions de repos stables.

9. Système selon la revendication 8, lorsqu'elle dépend directement ou indirectement de la revendication 6 ou de la revendication 7, dans lequel l'armature comporte une partie d'engagement de tubage (62) qui définit un profil dans un plan perpendiculaire à l'axe de pivotement, et le profil est conformé de manière à ce que le profil soit plus proche du socle lorsque l'armature occupe la première position que lorsque l'armature occupe la deuxième position, l'armature comporte de préférence une première partie d'engagement de socle qui engage le socle support lorsque l'armature occupe sa première position, la première partie d'engagement de socle comprend de préférence un premier galet (70), le premier galet définit de préférence partiellement le profil de la partie d'engagement de tubage de l'armature, l'armature comporte de préférence une deuxième partie d'engagement de socle (60) qui engage le socle support lorsque l'armature occupe sa deuxième position, et la deuxième partie d'engagement de socle comprend de préférence un deuxième galet (72).

10. Système selon la revendication 8 ou la revendication 9 comportant une cartouche amovible, dans lequel la cartouche comporte une longueur de tubage élastique (82) et le socle support (52) de la valve qui porte le tubage.

11. Système selon l'une quelconque des revendications 1 à 7, dans lequel la valve est une valve montée en ligne (400).

12. Système selon l'une quelconque des revendications précédentes comportant un tubage (98) de capteur de pression destiné à s'accoupler à un masque (96) d'un système à oxygène thérapeutique à une extrémité et couplé au capteur de pression (100) à l'autre extrémité.

13. Système selon l'une quelconque des revendications précédentes, dans lequel le régulateur d'alimentation en gaz (104) est configuré pour recevoir un signal de niveau d'oxygène depuis un capteur de saturation en oxygène (130).

14. Système selon la revendication 13, dans lequel le régulateur d'alimentation en gaz (104) est configuré pour générer des signaux de régulation pour ajuster la pression d'un gaz alimentant la valve.

15. Système selon la revendication 13 ou la revendication 14, lequel système comporte un affichage (114) et dans lequel le régulateur d'alimentation en gaz (104) et configuré pour générer un signal de fréquence de commutation fonction de la fréquence de commutation de la valve et délivrer le signal à l'affichage pour amener l'affichage à afficher une indication fonction du signal de fréquence de commutation.

FIG. 1

FIG. 2

FIG. 3

FIG. 4

FIG. 5

FIG. 6

FIG. 7

10

14

70    72        52

FIG. 8

34    12

FIG. 9

FIG. 10

FIG. 11

FIG. 12

FIG. 13

FIG. 14

FIG. 15

FIG. 16

FIG. 17

FIG. 18

FIG. 19

Torque

FIG. 20

Pinch Force

FIG. 21

FIG. 22

FIG. 23

FIG. 24

FIG. 25

FIG. 26

FIG. 27

## FIG. 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2466102 A
- US 4829947 A
- US 5351934 A
- FR 2865312
- US 4533890 A
- DE 19945262
- EP 0170894 A
- US 4910487 A
- EP 2945264 A
- GB 1590373 A
- US 6173711 B1